# NEUE EUROPÄISCHE PATENTSCHRIFT

(11) **EP 0 465 758 B2**
(45) Veröffentlichungstag und Bekanntmachung des Hinweises auf die Entscheidung über den Einspruch: **28.02.2001**
(45) Hinweis auf die Patenterteilung: 20.07.1994
(21) Anmeldenummer: 91101722.6
(22) Anmeldetag: 08.02.1991
(51) Int. Cl.: A61H 15/00

(54) **Massagegerät**
Massage device
Dispositif de massage

(30) Priorität: 11.07.1990 DE 9010455 U
(43) Veröffentlichungstag der Anmeldung: 15.01.1992
(73) Patentinhaber: Arnold, Gerhard, 65187 Wiesbaden (DE)
(72) Erfinder: Koll, Walter, D-5413 Bendorf/Rhein 3 (DE)
(74) Vertreter: Schlagwein, Udo, Dipl.-Ing.

(56) Entgegenhaltungen:
- EP-A- 0 092 449
- EP-A- 0 282 173
- EP-A- 0 346 942
- CH-A- 388 531
- DE-A- 3 313 893
- FR-A- 142 132
- FR-B- 843 978
- FR-B- 1 016 702
- US-A- 2 310 804

## Beschreibung

Die Erfindung betrifft ein Massagegerät mit einem Handgriff und zumindest einem zur Rollmassage dienenden, auf einer Rollkörperachse des Handgriffs in einem Gabelteil drehbar gelagerten Rollkörper, wobei die Rollkörperachse um eine senkrecht zur Rollkörperachse verlaufende Schwenkachse kippbar angeordnet ist und die Rollkörperachse seitlich des Handgriffes in dem durch die Schwenkachse schwenkbar mit dem Handgriff verbundenen Gabelteil angeordnet ist und die Schwenkachse mittig im Gabelteil und damit auch mittig zum Rollkörper verläuft.

Ein solches Massagegerät wurde auf einer Tagung vorgestellt. Vergleichbare Massagegeräte wurden druckschriftlich in der EP-0 346 942 und EP-A-0 282 173 beschrieben. Bei dem beschriebenen Massagegerät verläuft die Schwenkachse der Rollkörperachse auf der Mittellinie oder in der Mitteneben des Handgriffes zwischen dem Handgriff und dem Rollkörper. Da die Rollkörperachse in der Ebene des Handgriffes oder fluchtend zum Handgriff verläuft, hat das rollkörperseitige Ende des Handgriffes beim Massieren nur geringen Abstand von der Hautoberfläche. Dadurch besteht bei Benutzung des Massagegerätes die Gefahr, daß man mit dem schwenkachsenseitigen Ende des Handgriffes über die Haut reibt, was zu Verletzungen führen kann, zumindest aber unangenehm ist.

Ein weiterer Nachteil des bekannten Massagegerätes besteht darin, daß der Rollkörper beim Massieren eine feste Winkellage zum Handgriff hat. Man muß deshalb beim Rollen in Längsrichtung über gewölbte Hautpartien die Winkellage des Handgriffes verändern, damit der Rollkörper über einen möglichst großen Bereich aufliegt. Dieses Anpassen an den Hautverlauf erfordert beträchtliches Geschick und Aufmerksamkeit.

Der Erfindung liegt die Aufgabe zugrunde, ein Massagegerät der eingangs genannten Art so auszubilden, daß es ohne Verletzungsgefahr der Haut und auch bei stärker gekrümmten Körperpartien einfach und wirkungsvoll einzusetzen ist.

Diese Aufgabe wird erfindungsgemäß durch die Merkmale des kennzeichnenden Teils des Anspruchs 1 gelöst.

Ein solches Massagegerät eignet sich besonders zum Massieren gekrümmter Hautpartien.

Wenn mit diesem Massagegerät beispielsweise in Längsrichtung der Wirbelsäule gerollt wird, dann kippt der eine Rollkörper zur einen und der Rollkörper auf der anderen Wirbelsäulenseite zur anderen Seite, so daß beide Rollkörper über ihre volle Länge gegen die Haut anliegen.

Dank der erfindungsgemäßen Gestaltung eignet sich das Massagegerät ganz besonders zur Behandlung des Gesichtes, da dieses viele Wölbungen und Einbuchtungen aufweist, zum Beispiel die Augenhöhlen, die hervorstehenden Backenknochen, die Nase, das Kinn und die Lippenpartie. Durch die pendelnde Aufhängung des Rollkörpers wird auch bei längerer Massage eine Überbeanspruchung vorstehender Körperpartien vermieden.

Der Rollkörper kann sehr verschieden gestaltet sein. Er besteht vorzugsweise aus mehreren einzelnen Scheiben, welche auf ihrer Außenmantelfläche eine Verzahnung aufweisen, wie das in der FR-PS 843.987 beschrieben ist. Der Rollkörper kann auch statt einer Verzahnung nadelförmige Spitzen haben, wie sie in der DE-OS 32 21 750 gezeigt sind. Auch die in der DE-OS 36 10 220 gezeigte, schnekkenartige Ausbildung des Rollkörpers kann bei der vorliegenden Erfindung Anwendung finden.

Bei dem erfindungsgemäßen Massagegerät läßt sich die Schwenkbarkeit der Gabelteile auf einfache Weise durch Anschlagflächen der Gabelteile und Anlageflächen innerhalb der Ausnehmungen begrenzen.

Als besonders vorteilhaft für die Handhabung des Massagegerätes hat es sich erwiesen, wenn die Schwenkbarkeit der Gabelteile so begrenzt ist, daß die Rollkörper in einer Endstellung parallel zum Handgriff verlaufen und in ihrer anderen Endstellung mit dem rollkörperseitigen Ende des Handgriffes einen spitzen Winkel bilden.

Das Massagegerät kann wahlweise mit einem zu den Rollkörpern achsparallel ausgerichteten Handgriff oder mit einem quer zu ihnen ausgerichteten Handgriff benutzt werden, wenn die Gabelteile in einem Tragteil angeordnet sind und der Handgriff um eine senkrecht zu der Rollkörperachse verlaufende Achse schwenkbar mit dem Tragteil verbunden ist. Hierdurch kann man das erfindungsgemäße Massagegerät fallweise wie ein insgesamt gabelförmiges, beispielsweise in der FR-PS 843.978 beschriebenes Massagegerät benutzen.

Zur weiteren Verbesserung der Handhabung des Massagegerätes trägt es bei, wenn der Handgriff zusätzlich zur senkrecht zu den Rollkörperachsen verlaufenden Achse um eine parallel zu den Rollkörperachsen verlaufende Achse schwenkbar mit dem Tragteil verbunden ist.

Massagegeräte mit hintereinander angeordneten Rollkörpern werden in bestimmten Fällen bevorzugt, weil mit ihnen ein größerer Hautbereich gleichzeitig beeinflußt werden kann, als bei einem Massagegerät mit nur einem Rollkörper oder mehreren Rollkörpern nebeneinander.

Das Massagegerät kann zusätzlich zur mechanischen Hautbeeinflussung elektrisch auf die Haut einwirken, wenn der Rollkörper mit einer elektrischen Spannungsquelle verbunden ist. Durch den elektrischen Strom entsteht beim Massieren ein prickelndes Gefühl auf der Hautoberfläche. Muskulatur und Nerven werden dadurch angeregt.

Eine Steigerung des Massageeffektes läßt sich auch dadurch erreichen, daß der Rollkörper mit einem elektrischen Vibrator verbunden ist.

Das Massagegerät ist vom elektrischen Netz unabhängig, wenn zu seiner Spannungsversorgung im Handgriff eine Batterie angeordnet ist.

Die Erfindung läßt zahlreiche Ausführungsformen zu. Zwei davon sind in der beigefügten Zeichnung dargestellt und werden nachfolgend beschreiben. In ihr zeigen die
- Fig. 1: eine Seitenansicht eines erfindungsgemäßen Massagegerätes,
- Fig. 2: einen Längsschnitt durch das Massagegerät,
- Fig. 3: einen Querschnitt durch das Massagegerät entlang der Linie III - III in Fig. 2
- Fig. 4: einen Längsschnitt durch eine weitere Ausführungsform eines Massagegerätes,
- Fig. 5: eine teilweise geschnitten dargestellte Draufsicht auf das Massagegerät nach Fig.4.

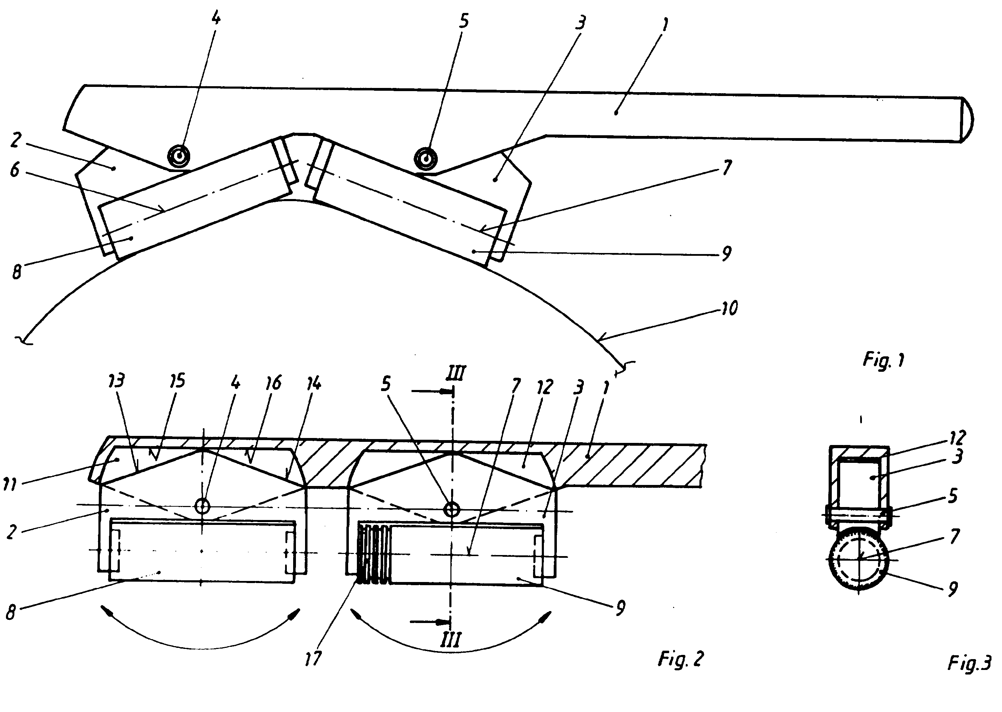

Das in Figur 1 dargestellte Massagegerät hat einen Handgriff 1, in welchem zwei Gabelteile 2, 3 jeweils um eine Schwenkachse 4, 5 pendelnd gehalten sind. Jedes Gabelteil 2, 3 lagert einen um eine Rollkörperachse 6, 7 drehbaren Rollkörper 8, 9. Diese Rollkörper 8, 9 berühren in der dargestellten Position eine konvex gewölbte Hautoberfläche 10. Sie sind um die Schwenkachsen 4, 5 derart zu verschwenken, daß sie sich aus der dargestellten Position über eine ausgerichtete Position bis in eine schräg ausgerichtete Position bewegen können.

Die Figur 2 läßt erkennen, daß im Handgriff zwei Ausnehmungen 11, 12 vorhanden sind, welche jeweils ein Gabelteil 2, 3 aufnehmen. Die Gabelteile 2, 3 haben im Inneren der jeweiligen Ausnehmung 11, 12 zwei in einem stumpfen Winkel aufeinanderstoßende Anschlagflächen 13, 14, welche bei einer Schwenkbewegung der Gabelteile 2, 3 gegen Anlageflächen 15, 16, die durch den Boden der Ausnehmung 11, 12 gebildet sind, anzuliegen vermögen. Dadurch wird die Schwenkbarkeit der Gabelteile 2, 3 begrenzt.

Für den Rollkörper 9 sind in Figur 2 einzelne Scheiben 17 skizziert, die um die Rollkörperachse 7 drehbar und auf ihrer Umfangsfläche gezahnt sein können, wie das beispielsweise in der FR-PS 843.978 beschrieben ist.

Die Figur 3 verdeutlicht die Gestaltung des Massagegerätes zusätzlich. Man erkennt das Gabelteil 3, welches in der Ausnehmung 12 um die Schwenkachse 5 pendelnd gehalten ist. Weiterhin ist der um die Rollkörperachse 7 drehbare Rollkörper 9 dargestellt.

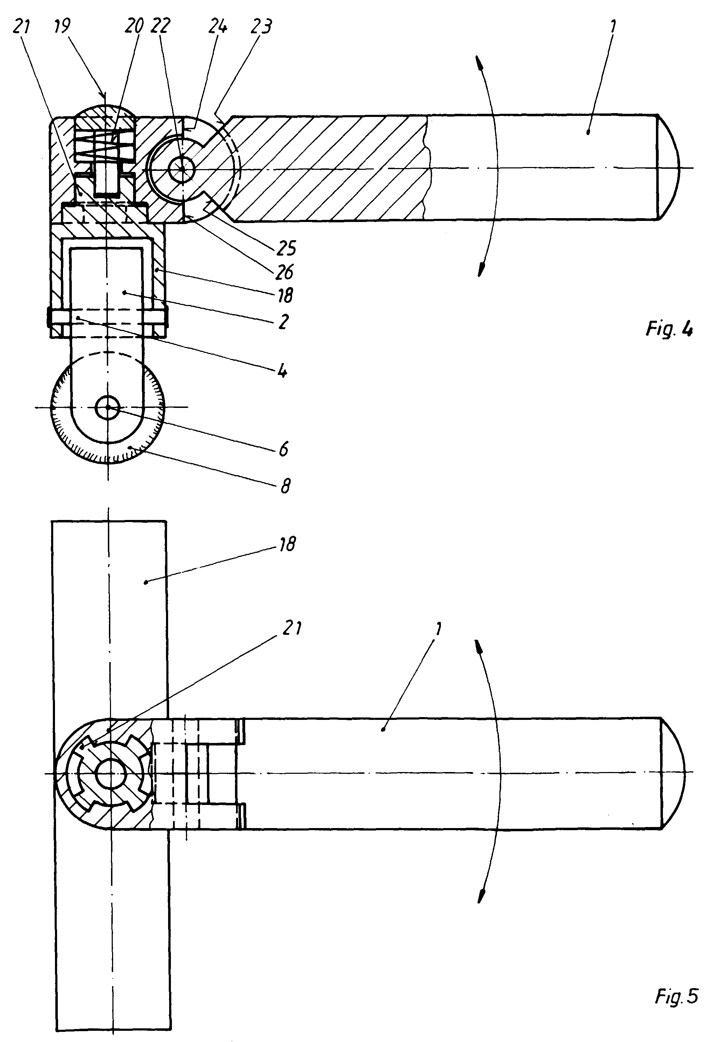

Bei der Ausführungsform nach Figur 4 ist ein Gabelteil 2 zu sehen, welches nicht unmittelbar in einem Handgriff 1, sondern in einem Tragteil 18 um die Schwenkachse 4 pendelnd gehalten ist. Mit diesem Tragteil 18 ist der Handgriff 1 um eine senkrecht Achse 19 verstellbar verbunden. Eine Druckfeder 20 hält in der dargestellten Stellung den Handgriff 1 in einer Verzahnung 21 des Tragteiles 18. Zieht man den Handgriff 1 relativ zum Tragteil 18 nach oben gegen die Kraft der Druckfeder 20, dann kommt es zu einem Ausrasten der Verzahnung 21. Man kann dadurch den Handgriff 1 um 90 Grad verdrehen und ihn anschließend erneut verrasten, indem man ihn losläßt. Die Druckfeder 20 drückt ihn dann wieder in die Verzahnung 21.

Die Figur 4 läßt weiterhin erkennen, daß der Handgriff 1 zusätzlich um eine quer zur Achse 19 verlaufende Achse 22 verschwenkbar ist. Diese Verschwenkbarkeit wird durch Anschlagflächen 23, 24, 25, 26 nach beiden Seiten hin begrenzt.

Die teilweise geschnitten dargestellte Draufsicht gemäß Figur 5 zeigt die Verzahnung 21 des Tragteiles 18, durch die der Handgriff 1 in der quer zum Tragteil 18 verlaufenden Stellung und in einer zu ihm fluchtenden Stellung fixierbar ist. Zusätzlich ist die horizontal verlaufende Achse 22 zu sehen, die die weitere Verschwenkbarkeit des Handgriffes 1 ermöglicht.

### Auflistung der verwendeten Bezugszeichen

- 1: Handgriff
- 2: Gabelteil
- 3: Gabelteil
- 4: Schwenkachse
- 5: Schwenkachse

- 6: Rollkörperachse
- 7: Rollkörperachse
- 8: Rollkörper
- 9: Rollkörper
- 10: Hautoberfläche

- 11: Ausnehmung
- 12: Ausnehmung
- 13: Anschlagfläche
- 14: Anschlagfläche
- 15: Anlagefläche

- 16: Anlagefläche
- 17: Scheibe
- 18: Tragteil
- 19: Achse
- 20: Druckfeder

- 21: Verzahnung
- 22: Achse
- 23: Anschlagfläche
- 24: Anschlagfläche
- 25: Anschlagfläche

- 26: Anschlagfläche

## Patentansprüche

1. Massagegerät mit einem Handgriff (1) und zumindest einem zur Rollmassage dienenden, auf einer Rollkörperachse (6, 7) des Handgriffs (1) in einem Gabelteil (2, 3) drehbar gelagerten Rollkörper (8, 9), wobei die Rollkörperachse (6, 7) um eine senkrecht zur Rollkörperachse (6, 7) verlaufende Schwenkachse (4, 5) kippbar angeordnet ist und die Rollkörperachse (6, 7) seitlich des Handgriffes (1) in dem durch die Schwenkachse (4, 5) schwenkbar mit dem Handgriff (1) verbundenen Gabelteil (2, 3) angeordnet ist und die Schwenkachse (4, 5) mittig im Gabelteil (2, 3) und damit auch mittig zum Rollkörper (8, 9) verläuft, **dadurch gekennzeichnet**, daß der Handgriff (1) fluchtend hintereinander zwei Gabelteile (2, 3) mit jeweils einem Rollkörper (8, 9) aufweist.

2. Massagegerät nach Anspruch 1, **dadurch gekennzeichnet**, daß die Gabelteile (2, 3) jeweils in eine zu einer Seite hin offene Ausnehmung (11, 12) des Handgriffes (1) eingesetzt und die Schwenkachse (4, 5) jeweils durch zwei die Ausnehmung (11, 12) begrenzende Seitenteile des Handgriffes (1) geführt ist.

3. Massagegerät nach Anspruch 1 oder 2, **dadurch gekennzeichnet**, daß die Schwenkbarkeit der Gabelteile (2, 3) durch Anschlagflächen (13, 14) der Gabelteile (2, 3) und Anlageflächen (15, 16) innerhalb der Ausnehmungen (11, 12) begrenzt ist.

4. Massagegerät nach Anspruch 3, **dadurch gekennzeichnet**, daß die Schwenkbarkeit der Gabelteile (2, 3) so begrenzt ist, daß die Rollkörper (8, 9) in einer Endstellung parallel zum Handgriff (1) und in seiner anderen Endstellung mit dem rollkörperseitigen Ende des Handgriffes (1) einen spitzen Winkel bilden.

5. Massagegerät nach zumindest einem der vorangehenden Ansprüche, **dadurch gekennzeichnet**, daß die Gabelteile (2, 3) in einem Tragteil (18) angeordnet sind und der Handgriff (1) um eine senkrecht zu der Rollkörperachse (6) verlaufende Achse (19) schwenkbar mit dem Tragteil (18) verbunden ist.

6. Massagegerät nach Anspruch 5, **dadurch gekennzeichnet,** daß der Handgriff (1) zusätzlich zur senkrecht zu der Rollkörperachse (6) verlaufenden Achse (19) um eine parallel zu der Rollkörperachse verlaufende Achse (22) schwenkbar mit dem Tragteil (18) verbunden ist.

7. Massagegerät ach zumindest einem der vorangehenden Ansprüche, **dadurch gekennzeichnet**, daß die Rollkörper (8, 9) mit einer elektrischen Spannungsquelle verbunden sind.

8. Massagegerät nach zumindest einem der vorangehenden Ansprüche, **dadurch gekennzeichnet**, daß die Rollkörper (8, 9) mit einem elektrischen Vibrator verbunden sind.

9. Massagegerät nach den Ansprüchen 7 oder 8, **dadurch gekennzeichnet**, daß zu seiner Spannungsversorgung im Handgriff (1) eine Batterie angeordnet ist.

## Claims

1. Massage device with a handle (1) and at least one roller (8, 9) rotatably mounted in a fork member (2, 3) on a roller axis (6, 7) of the handle (1) and serving for rolling massage, the roller axis (6, 7) arranged so as to tilt round a pivot axis (4, 5) extending perpendicularly to the roller axis (6, 7)and the roller axis (6, 7) arranged to the side of the handle (1) in the fork member (2, 3) pivotally connected to the handle (1) by means of the pivot axis (4, 5) and the pivot axis (4, 5) extends centrally in the fork member (2, 3) and therefore also centrally relative to the roller (8, 9), characterised in that the handle (1) has, arranged one behind the other in alignment, two fork members (2, 3) with a respective roller (8, 9).

2. Massage device according to claim 1, characterised in that the fork members (2, 3) are each inserted into a recess (11, 12) in the handle (1) which is open on one side and the pivot axis (4, 5) are each guided through two lateral parts of the handle (1) limiting the recess (11, 12).

3. Massage device according to claim 1 or 2, characterised in that the pivotability of the fork members (2, 3) is limited by stop faces (13, 14) of the fork members (2, 3) and contact faces (15, 16) within the recesses (11, 12).

4. Massage device according to claim 3, characterised in that the pivotability of the fork members (2, 3) is limited such that the rollers (8, 9) form an acute angle in an end position parallel to the handle (1) and in their other end position with the rollerside end of the handle (1).

5. Massage device according to at least one of the preceding claims, characterised in that the fork members (2, 3) are arranged in carrying member (18) and the handle (1) is connected pivotally to the carrying member (18) round an axis (19) extending perpendicularly to the roller axis (6).

6. Massage device according to claim 5, characterised in that, in addition to the axis (19) extending perpendicularly to the roller axis (6), the handle (1) is connected pivotally to the carrying member (18) round an axis (22) extending parallel to the roller axis.

7. Massage device according to at least one of the preceding claims, characterised in that the rollers (8, 9) are connected to an electric voltage source.

8. Massage device according to at least one of the preceding claims, characterised in that the rollers (8, 9) are connected to an electric vibrator.

9. Massage device according to claims 7 or 8, characterised in that a battery is arranged in the handle (1) for supplying a voltage to the massage device.

## Revendications

1. Appareil de massage avec une poignée (1) et au moins un corps roulant (8, 9) servant au massage et monté à rotation sur un axe (6, 7) de la poignée et dans une fourche (2, 3), l'axe (6, 7) de corps roulant est retournable autour d'un axe d'oscillation (4, 5) s'étendant perpendiculairement à l'axe (6, 7) de corps roulant et l'axe (6, 7) de corps roulant est disposé sur un coté de la poignée (1), dans la fourche (2, 3) montée de façon oscillante dans la poignée (1), et un axe d'oscillation (4, 5) s'étend de façon centrale dans la fourche (2, 3) et aussi par rapport au corps roulant (8, 9), caractérisé en ce que la poignée (1) comporte deux fourches (2, 3) alignées l'une derrière l'autre avec chacune un corps roulant (8, 9).

2. Appareil de massage selon la revendication 1, caractérisé en ce que les fourches (2, 3) sont montées chacune dans un évidement (11, 12) débouchant sur un coté de la poignée (1), et en ce que l'axe d'oscillation (4, 5) est porté et limité chacun par deux parties latérales des évidements (11, 12).

3. Appareil de massage selon l'une des revendications 1 ou 2, caractérisé en ce que le basculement des fourches (2, 3) est limité par des surfaces de butées (13, 14) des fourches (2, 3) et des surfaces (15, 16) internes des évidements (11, 12).

4. Appareil de massage selon la revendication 3, caractérisé en ce que le basculement des fourches (2, 3) est limité de telle façon que les corps roulants (8, 9) dans une position extrême, sont parallèles à la poignée (1), et dans une autre position extrême formes un angle aigu avec l'extrémité de la poignée (1) disposée sur les cotés des corps roulants.

5. Appareil de massage selon au moins l'une des revendications précédentes, caractérisé en ce que les fourches (2, 3) sont disposées dans une pièce support (18) et en ce que la poignée (1) est montée de façon oscillante par rapport à la pièce support (18) autour d'un axe (19) s'étendant perpendiculairement à l'axe (6) des corps roulants.

6. Appareil de massage selon la revendication 5, caractérisé en ce que la poignée (1), montée de façon oscillante par rapport à la pièce support (18) autour d'un axe (19) perpendiculaire à l'axe (6) des corps roulants, est en outre montée oscillante par rapport à la pièce support (18) autour d'un axe (22) parallèle à l'axe des corps roulants.

7. Appareil de massage selon au moins l'une des revendications précédentes, caractérisé en ce que les corps roulants (8, 9) sont reliés à une source de tension électrique.

8. Appareil de massage selon au moins l'une des revendications précédentes, caractérisé en ce que les corps roulants (8, 9) sont reliés à un vibrateur électrique.

9. Appareil de massage selon l'une des revendications 7 à 8, caractérisé en ce qu'une batterie est disposée dans la poignée (1) pour l'alimentation en tension de l'appareil.
